# EUROPEAN PATENT APPLICATION

(11) **EP 1 552 849 A1**
(43) Date of publication of application: **13.07.2005**
(21) Application number: 03797657.8
(22) Date of filing: 18.09.2003
(51) Int. Cl.: A61K 45/00, A61K 45/06, A61P 35/00, A61P 43/00

(54) **IMMUNOTHERAPEUTIC FOR CANCER**

(30) Priority: 19.09.2002 JP 2002273738; 26.09.2002 JP 2002281780; 06.12.2002 JP 2002354515; 05.06.2003 JP 2003161238; 13.06.2003 JP 2003169153
(71) Applicant: Orient Cancer Therapy Co. Ltd, Mitaka-shi, Tokyo 181-0015 (JP)
(72) Inventor: YAGITA, Akikuni, Mitaka-shi, Tokyo 181-0015 (JP)
(74) Representative: De Clercq, Ann
(86) International application number: PCT/JP2003/011895
(87) International publication number: WO 2004/026341

(57) **Abstract**

There is provided means for increasing complete remission rates and shortening a period to complete remission and for achieving a synergistic effect with immunotherapy that is directed at bringing about enhanced effects from molecule-targeting therapeutic drugs. More specifically, an object is to achieve a synergistic effect by combining use of a novel immunotherapy for cancer that focuses on CTL activity, NKT activity, NK activity and -VEGF and the like, and molecule-targeting therapeutic drugs, particularly tyrosine kinase inhibitors. The present invention was accomplished based on the finding that combined use of a tyrosine kinase inhibitor and an IL-12 inducer achieves a superior synergistic effect in cancer therapy.

## Description

This application claims the benefit of priority from Japanese Patent Application Nos. 2002-273738, 2002-281780, 2002-354515, 2003-161238, and 2003-169153, which are incorporated herein by reference.

### Technical Field

The present invention provides a new area of cancer therapy. More specifically, the present invention relates to providing a novel therapeutic agent for cancer by combining use of tyrosine kinase inhibitors that are attracting attention as a novel cancer therapy and a novel immunotherapy for cancer developed by Akikuni Yagita MD, that focuses on the kinetics of NK cell activating capabilities, NKT cell activating capabilities, neovascularization inhibiting capabilities, IL-12 production inducing capabilities, and IFNγ production inducing capabilities.

### Background Art

In selecting substances that are useful for prevention or treatment of malignant neoplasms (cancer), emphasis has hitherto been placed on their direct effect on cancer cells. While immunostimulators have been recognized as useful for cancer treatment, all compounds obtained as immunostimulators are feeble in their anticancer effect, leaving a sufficient cancer treatment effect unattained both by immunotherapy alone and by a combination of immunotherapy and chemical therapy.

The present inventor, Yagita MD, noting previously the usefulness of substances inducing interleukin 12 (IL-12) in vivo as a breakthrough method in cancer treatment, discovered that processed mushroom mycelium has that function, and thus established a cancer treatment method that might be described as "novel immunotherapy for cancer" (NITC) . Heretofore, although it was known that IL-12 has an anti-cancer effect, IL-12 has been unusable as an anticancer drug, because of the fact that patients are unable to endure treatment due to its side effects when IL-12 itself is directly administered in vivo. However, the preparation containing the processed mushroom mycelium reported by Yagita achieved outstanding curing and life-prolonging effects in cancer treatment. That is, Yagita achieved the purpose of cancer treatment by administering the processed mushroom mycelium in effective amounts sufficient to induce IL-12 in vivo [(Patent Literature 1) Japanese Patent Laid-Open No. 1998-139670].

The IL-12 has activating and augmenting effects on killer T cell through the route of TNFα → IFNγ → IL-12 → CTL activation. That is, augmentation of IL-12 production holds promise of an anticancer effect by activating and augmenting killer T cell.

Yagita also reported, aside from the system of IL-12 production augmentation, that NKT cell activation is useful for anticancer effect. Taniguchi et al. discovered a specific glycolipid antigen recognized by a specific T cell antigen receptor (TCR), Vα24Vβ11, carried in NKT cell, and reported that this antigen is α-galactosylceramide. Further, they proved that, in a cancer-bearing mouse administered with α-galactosylceramide, NKT cell is activated and metastasis suppressed, although the cancer disappearance is not observed.

It is reported that NK cell antigen receptor (NKR-P1: natural killer receptor P1) is present as another receptor in NKT cell [(Non-patent Literature 1) Tokushuu NKT Saibou no Kiso to Rinshou: Saishin Igaku, Vol. 55, No. 4, 2000, p. 818-823].

Yagita found that NKR-P1 also participates in NKT cell activation and that this activation enhances anticancer effect [(Patent Literature 2) US 2002-0010149Al].

Molecule-targeting therapeutic agents for cancer are attracting attention in their significance as a new type of anticancer drug compared with the conventional cell-targeting therapeutic agents. Among these, in particular, tyrosine kinase inhibitors are attracting attention as drugs that have an inhibitory action for signal transduction. ZD1839 (registered trademark IRESSA; AstraZeneca) has an action that competes with ATP in ATP-binding site of EGFR (epidermal growth factor receptor) tyrosine kinase, and suppresses tyrosine kinase activity by suppressing autophosphorylation of tyrosine kinase. As a result, it expresses an anticancer action by blocking signal transduction of EGFR (EGFR tyrosine kinase that exists in the intracellular domain is activated by the binding of a ligand such as epidermal growth factor (EGF) to the extracellular domain of EGFR, thereby inducing autophosphorylation of EGFR and phosphorylation of various intracellular target proteins to transduce a proliferation signal from the cell surface to the nucleus, and transduce a proliferation signal from the cancer cell surface to the nucleus to cause proliferation, invasion, metastasis and neovascularization of cancer cells) that is related to proliferation, invasion, differentiation and metastasis. IMC-C225 (EGFR-targeting monoclonal antibody) recognizes an EGFR receptor site on the cell membrane surface and inhibits tyrosine kinase activity by suppressing autophosphorylation of EGFR. Herceptin is a monoclonal antibody for Her2/Neu that has homology with EGFR, and STI-571 (Gleevec) can inhibit tyrosine kinase activity of BCR-Abl as well as tyrosine kinase activity of c-kit [(Non-patent Literature 2) Ketsueki Meneki Shuyou, Vol. 7, No. 3, 2002-7].

Although these kinds of molecule-targeting therapeutic agents are attracting attention as therapeutic agents for cancer that have a new mechanism, the effects thereof can not yet be called revolutionary. For example, ZD1839 (IRESSA) is a potent and selective EGFR tyrosine kinase inhibitor that was newly developed by AstraZeneca, and the usefulness thereof has also been demonstrated in humans. However, in clinical results thereof for non-small-cell lung cancer, prostatic cancer and the like, PR (partial remission) was found in 10 to 20-odd % of the cases, and almost no CR (complete remission) was found. Complete remission in extremely rare cases took as long a period as 4 months or more to reach. Thus, although combined therapy using ZD1839 (IRESSA) and various anticancer agents is being tried, currently no synergistic effect or additive effect has been obtained.

### Disclosure of the Invention

An object of the present invention is to produce a more advantageous effect from the aforementioned kinds of molecule-targeting therapeutic drugs and to provide means for increasing complete remission rates, shortening periods until complete remission and achieving a synergistic effect with immunotherapy. More specifically, an object of the invention is to achieve a synergistic effect by combining use of a novel immunotherapy for cancer that focuses on CTL activation, NKT activity, NK activity and -VEGF and the like, together with molecule-targeting therapeutic drugs, in particular tyrosine kinase inhibitors.

The present invention has been completed based on the discovery that combined use of a tyrosine kinase inhibitor and an IL-12 inducer achieves a superior synergistic effect in cancer therapy.

That is, the present invention comprises the following.
1. A therapeutic agent for cancer, wherein a tyrosine kinase inhibitor and an IL-12 inducer are used in combination.
2. The therapeutic agent for cancer according to the above 1, wherein the tyrosine kinase inhibitor has a target-selective action for at least one receptor selected from the group consisting of the following 1) to 7): 1) HER2/neu; 2) HER3; 3) HER4; 4) c-kit; 5) PDGFR; 6) bcr-abl; and 7) EGFR.
3. The therapeutic agent for cancer according to the above 1, wherein the tyrosine kinase inhibitor has an action with EGFR or c-kit selectively targeted.
4. The therapeutic agent for cancer according to any one of the above 1 to 3, wherein the IL-12 inducer is a substance having a β1,3/1,6 glucan structure.
5. The therapeutic agent for cancer according to the above 4, wherein the IL-12 inducer is an ingredient derived from mushroom mycelium or a yeast-derived ingredient that has a β1,3/1,6 glucan structure.
6. The therapeutic agent for cancer according to any one of the above 1 to 5, which is used in no combination with a chemotherapeutic agent for cancer and a radiation therapy.
7. The therapeutic agent for cancer according to any one of the above 1 to 6, which is used in combination with a substance that selectively acts on NKR-P1 of NKT cell to cause activation of NKT cell.
8. The therapeutic agent for cancer according to any one of the above 1 to 7, which is used in combination with a substance having neovascularization inhibiting capabilities.
9. The therapeutic agent for cancer according to any one of the above 1 to 8, wherein a treatment that combines use of a tyrosine kinase inhibitor and an IL-12 inducer is carried out employing either one of the following 1) and 2) as a marker:
   1) an NKTP value before administration showing a measurement value of 5% or more;
   2) a Th2 value before administration showing a measurement value of 3% or more.
10. The therapeutic agent for cancer according to any one of the above 1 to 9, wherein a Th1/Th2 ratio that shows an increased measurement value after several months of administration of IRESSA in comparison to a value before the administration is taken as a marker for continuation of the combined treatment.
11. The therapeutic agent for cancer according to the above 10, wherein an NKTP value before administration shows a measurement value below 5%.
12. The therapeutic agent for cancer according to the above 9, wherein measurement values of IL-12 and INFγ that have not decreased after several months of administration of IRESSA in comparison with the measurement values before the administration are taken as markers for continuation of the combined treatment.
13. The therapeutic agent for cancer according to any one of the above 1 to 12, wherein the therapeutic agent for cancer is a therapeutic agent for pulmonary adenocarcinoma.
14. A therapeutic method for cancer that uses the therapeutic agent for cancer according to any one of the above 1 to 13.

### Brief Description of the Drawings

Figure 1 is a view of an x-ray radiograph of a patient.
Figure 2 is a view of an x-ray radiograph of a patient.
Figure 3 is a view illustrating essential signals for osteoclast differentiation.
Figure 4 shows the cases of effective treatment of pulmonary (adeno)carcinoma.
Figure 5 shows the cases of effective treatment of cancer of the large intesinal.
Figure 6 shows the cases of effective treatment of various kinds of cancer.
Figure 7 illustrates the level of contribution of each marker with respect to effective treatment determined by logistic regression coefficient analysis (pulmonary adenocarcinoma).
Figure 8 illustrates the level of contribution of each marker with respect to effective treatment determined by logistic regression coefficient analysis for patients administered with IRESSA (pulmonary adenocarcinoma).
Figure 9 shows a comparison of effective cases (B group) and ineffective cases (A group) prior to IRESSA administration.
Figure 10 shows a comparison of effective cases (B group) and ineffective cases (A group) for NKTP<5.0.
Figure 11 shows effective and ineffective cases according to two threshold values.
Figure 12 shows a difference in acting period between IRESSA and the novel immunotherapy for cancer.
Figure 13 shows a comparison between C group and D group prior to IRESSA administration.
Figure 14 illustrates differences in cytokines between C group and D group.
Figure 15 illustrates a hypothesis concerning the mechanism of synergistic action of IRESSA and NITC in an antitumor action.

### Detailed Description of the Invention

The present invention is explained in further detail below, and the technical and scientific terms used herein have the meanings as normally understood by those having an ordinary knowledge in the technical field to which this invention pertains unless otherwise defined.

The novel immunotherapy for cancer (NITC) of Dr. Yagita MD, the present inventor, is a therapeutic procedure comprising a combination of four different action mechanisms.

The first action mechanism is a method directed at reducing cancer by administering a neovascularization inhibitor (Better Shark) to block blood flow to the cancer. The effect of this method can be assessed by measuring the vascular endothelial growth factor (VEGF). An inhibitory action for neovascularization can be determined by a minus value (-VEGF) for the VEGF value. It is also possible to assess neovascularization inhibiting capabilities by use of values for other vascular growth factors such as FGF or HGF in place of the VEGF value. Further, the inhibitory capabilities can also be assessed by a positive value of a neovascularization inhibitor in place of VEGF (for example, an endostatin value).

The second action mechanism is a method that activates CTL by administration of a compound having a β1,3 glucan structure to induce Th1 cytokines (TNFα, IFNγ, IL-12). Although CTL activation can be judged by CD8(+) perforin production capability, this CD8(+) perforin value includes those represented by cytotoxic T cell (CTL) and immunosuppressive T cell (STC: suppressor T cell), of which the former impairs cancer cell, whereas the latter is activated to ultimately result in cancer growth. Accordingly, evaluation cannot be conducted simply with its absolute value. However, if IFNγ is of 10 IU/ml or more or if IL-12 is of 7.8 pg/ml or more, the value is judged as being represented by CTL, and if IFNγ and IL-12 are low, the value is judged as being represented by STC. Therefore, CTL activation can be evaluated using IFNγ production capability (IFNγ value) or IL-12 production capability (IL-12 value).

Effector cells that are activated by administration of compounds having an α1,3 glucan structure for the third and fourth action mechanisms are NK cell and NKT cell. Both NK and NKT cells carry NKR-P1 (NK cell receptor CD161 (+)). For the former, NK cell count can be measured by the CD3(-)CD161(-) surface marker, and its activation can be judged by the CD3(-)CD161(+) perforin production capability. For the latter, on the other hand, NKT cell count can be measured by the CD3(+)CD161(+), and NKT cell activation can be measured by its perforin production capability (represented by "NKTP").

In cancer treatment, therefore, it is possible to evaluate effector cell or inhibitory action for neovascularization using the measurement items given below in novel immunotherapy for cancer (NITC) and common immunotherapies alike. More specifically, CTL activation can be evaluated by IFNγ or IL-12 production inducing capability. NK cell activation can be evaluated by CD3(-)CD161(+) or CD3(-)CD161(+) perforin value. NKT cell activation can be evaluated by CD3(+)CD161(+) or CD3(+)CD161(+) perforin value (NKTP value).

This invention was accomplished by examining clinical results obtained by combining use of tyrosine kinase inhibitors with the aforementioned novel immunotherapy for cancer. As a novel immunotherapy for cancer (NITC), the present inventor combined use of a compound having an α1,3 glucan structure, a compound having a β1, 3 glucan structure and a substance having an inhibitory action for neovascularization (shark cartilage) for administration to cancer patients, and measured various cytokines such as IL-12 and IFNγ. In this connection, for CD8(+) perforin production, a strong positive correlation existed with production of IFNγ and IL-12, and it was thus found that measurement of CD8(+) perforin production is significant for evaluating a route of CTL activation.

Because of this significance, determination of CD8(+) perforin production capability can be applied to a method for screening for useful CTL activators (i.e., IL-12 inducers), and utilization of this screening method enables identification of a novel β1,3 glucan having CTL activation capability (IL-12 production inducibility). An IL-12 inducer used in this invention is not particularly limited, and a broad range of inducers can be used. For example, a mushroom mycelium composition having a β1,3 glucan structure (for example, the following products: ILX (trade name), available from Tozai Iyaku Kenkyusho, Ltd.; ILY (trade name), available from Seishin Enterprise Co., Ltd.; and AHCC, available from K. K. Amino Up), or various kinds of yeasts that have a β1,3 glucan structure (marine yeast, baker's yeast, or NBG™) can be utilized. Further, for a novel IL-12 inducer, a person skilled in the art can readily identify an IL-12 inducer (CTL activator) by combining measurement of CD8 perforin production capability. The term "CTL activator" has the same meaning as an IL-12 inducer as used herein.

In this invention, combined use of the IL-12 inducer and a tyrosine kinase inhibitor is essential. Although ZD1839 (trade name IRESSA) or STI571 (trade name Gleevec) are used in specific examples, various kinds of tyrosine kinase inhibitors can be effectively utilized herein. Examples of target molecules of these include HER2/neu, HER3, HER4, c-kit, PDGFR, bcr-abl, and EGFR. The most effective molecule is EGFR or c-kit.

Although a dosage of a tyrosine kinase inhibitor will be in accordance with a recommended dosage of the respective molecule-targeting compounds, an oral administration of 10 to 500 mg/day may be carried out.

The combined use of an IL-12 inducer and a tyrosine kinase inhibitor may be administered at an early stage of treatment, and either thereof may be administered to precede the other, but it is not particularly limited. In a specific example, combined use of a tyrosine kinase inhibitor was commenced after a specified period of administration of an NITC therapy, particulary an IL-12 inducer, and dramatic clinical effects were confirmed.

In the present invention, in addition to an IL-12 inducer, combined use of an NK activator or an NKT activator is possible. A composition comprising a compound having an al,3 glucan structure such as a nigerooligosaccharide or a fucoidan is useful as an NK activator or an NKT activator. Various compounds that have an α1,3 glucan structure are known, and a person skilled in the art can readily identify an NK activator or NKT activator by combining this known structure with measurement of CD3(-)CD161(+), CD3(-)CD161(+) perforin production capability, CD3(+)CD161(+), or CD3(+)CD161(+) perforin production capability. In this connection, the term "CD3(+)CD161(+)" refers to as acting on receptor NKR-P1 of NKT cell.

As examples of a saccharide substance having an α1,3 glucan structure, a nigerooligosaccharide (TSO), a fucoidan, an oligosaccharide sulfate and the like may be mentioned.

A nigerooligosaccharide is a saccharide containing 3-O-α-D-glucopyranosil-D-glucose as a constitutional unit. Typical examples thereof include nigerose, nigerosylglucose and nigerosylmaltose.

Further, as a commercially available nigerooligosaccharide, nigerooligosaccharides-supplemented liquid sugar syrup (available from Takeda Food Products, Ltd.) may be mentioned, and the main nigerooligosaccharides contained therein are (1) nigerose α-D-Glcp-(1,3)-D-Glc; (2) nigerosylglucose α-D-Glcp-(1,3)-α-D-Glcp-(1,4)-D-Glc; and (3) nigerosylmaltose α-D-Glcp-(1,3)-α-D-Glcp-(1,4)-α-D-Glcp-(1,4)-D-Glc (where Glc is an abbreviation for glucose, and p is an abbreviation for pyranose).

In a strict sense a fucoidan is a polysaccharide containing sulfated fucose in which one sulfuric acid molecule is bound to 2 to 6 molecules of fucose, and a fucoidan-like polysaccharide which comprises this substance and xylose or uronic acid is called "fucoidan" at the food level. Fucoidin is prepared, for example, by crushing tangle weed and forming the ground product into chips, extracting water-soluble components therefrom, removing the residue after extraction by centrifugation and then removing low-molecular substances such as iodine or sodium chloride by ultrafiltration, followed by freeze-drying.

Examples of fucoidan include phaeophyte-derived fucoidan, such as fucoidan derived from *Kjellmaniae crassifolia,* and fucoidan derived from Okinawa mozuku. At least three kinds of fucoidan are derived from *phaeophyte laminariaceae* such as *Kjellmaniae crassifolia*, these are F-fucoidan (a polymer of α-L-fucose), U-fucoidan (having β-D-glucuronic acid and α-D-mannose as a main chain, and α-L-fucose as a side chain) and G-fucoidan (having β-D-galactose as a main chain, and α-L-fucose as a side chain), and fucose is sulfated in each of these kinds of fucoidan.

As an oligosaccharide sulfate, for example, an extract derived from susabinori (*Porphyra yezoensis*) manufactured by Shirako Co., Ltd. may be mentioned. The main components of the extract are an oligosaccharide of galactan sulfate comprising an α1,3 bond and an oligosaccharide of galactan sulfate comprising an al, 3 bond and a β1,4 bond.

The combined use of a tyrosine kinase inhibitor and a CTL activator (IL-12 inducer or INFγ inducer) according to this invention, and use thereof further combined with an NK activator, an NKT activator or a neovascularization inhibitor are effective for treatment of lung cancer (pulmonary squamous cell carcinoma, pulmonary adenocarcinoma, small cell lung cancer), thymoma, thyroid cancer, prostate cancer, renal cancer, bladder cancer, colon cancer, cancer of the rectum, cancer of the esophagus, cancer of the cecum, ureteral cancer, breast cancer, uterine cervix cancer, brain cancer, lingual cancer, pharyngeal cancer, nasal cancer, laryngeal cancer, gastric cancer, hepatic cancer, bile duct cancer, testicular carcinoma, ovarian cancer, uterine body cancer, metastatic bone cancer, malignant melanoma, osteosarcoma, malignant lymphoma, plasmacytoma and liposarcoma, by selecting a dosage form thereof.

The combined use of a tyrosine kinase inhibitor and a CTL activator (IL-12 inducer or INFγ inducer) according to this invention, and use thereof further combined with an NK activator, an NKT activator, or a neovascularization inhibitor, may be employed in a prescription that can induce or enhance activation thereof and further maintain the activation. That is, an administration period or a dosage that can induce or enhance activation thereof and further maintain the activation may be selected to employ. More specifically, the dosage for a compound having an α1,3 glucan structure that is an NK activator or NKT activator is approximately 1 to 40 g per day, preferably approximately 5 to 20 g per day, and the dosage for a compound having a β1,3 glucan structure that is a CTL activator (IL-12 inducer or INFγ inducer) is approximately 1 to 10 g per day, preferably approximately 3 to 6 g per day. An administration period is generally between 10 days and 24 months, and the frequency of administration is alternate days or 1- to 3-times per day, and preferably administration is carried out everyday. The CTL activator (IL-12 inducer or INFγ inducer), NK activator and NKT activator are preferably orally ingested. Naturally, they can be parenterally ingested (including intravenous and intramuscular administration) by reducing the dosage and preparing them to a quality for allowing parenteral ingestion.

When therapy is conducted that uses an anticancer (chemotherapy) agent, radiation or steroid-combined therapy in addition to the combined treatment of the present invention, of the two kinds of immune systems, the system TNFα → IFNγ → IL-12 → killer T-cells is noticeably impaired. Therefore, these are preferably not used in this invention. However, when administering an antineoplastic agent, application of an administration method such as low dose chemotherapy that is a method that does not obstruct the aforementioned immune system, more specifically, a method administering a low dose of 5FU, UFT, mifurol, furtulon, or CDDP (5 µg to 10 µg), or taxotere or low-dose antineoplastic agents such as taxol, adriamycin, mitomycin or CPT-11, is useful. It is similarly necessary to select application of low dose irradiation in radiation therapy and low dose administration in steroid therapy or the like.

Measurement methods for cells and individual cytokines are exemplified hereunder.

### (NKT Cell Measurement) (NK Cell Measurement) (CD8 Measurement)

Measurement of NKR-P1-bearing NKT cell can be conducted by measuring cell surface antigens (CD3 and CD161) existing specifically on the NKT cell surface. More specifically, peripheral blood lymphocytes are examined with respect to cells with positive CD3 and positive CD161 (CD3+CD161+). That is, CD3 and CD161, NKT cell surface antigens, are measured by the two-color assay through flow cytometry using monoclonal antibody. Here, the term "NKT cell activation" refers to as the CD3+CD161+ NKT cell proportion in lymphocyte being 10% or more and preferably 16% or more. The term "NKT cell activating capability" denotes a capability of increasing the NKT cell proportion to 10% or more and preferably 16% or more, or a capability of further augmenting the NKT cell proportion from a proportion before administration of a certain substance.

Likewise, the term "(CD3-CD161+)" refers to examination of cells with negative CD3 and positive CD161. This method is useful for NK cell measurement.

Further, the term "CD8+" denotes examination of cells with positive CD8. This method is useful for CTL activation measurement.

In the examples, using blood obtained from cancer patients, blood cells were discriminated as positive or negative of the cell surface antigens CD3, CD161 and CD8, with each cell proportion measured according to a conventional method by two-color assay using flow cytometry. At this time, for respective monoclonal antibodies to CD3, CD161 and CD8, those manufactured by Coulter or Becton Dickinson were used.

### (Perforin Production Cell Measurement)

Two among CD3, CD161 and CD8, cell surface antigens, and perforin are measured with respect to peripheral blood lymphocyte according to a conventional method by three-color assay using flow cytometry. More specifically, a fixative is added to sampled blood, thus fixing cells. After addition of membrane permeating solution, anti-perforin antibody (manufactured by Pharmingen) is added for reaction. Further, PRE-Cy5 labeled second antibody (manufactured by DAKO) is added for reaction, followed by addition of anti-CD3-PE (Coulter 6604627) antibody and anti-CD161-FITC (B-D) antibody for reaction, after which measurement is conducted by flow cytometry. In the drawings and tables, the abbreviation "PER" is used.

### (Sample Preparation for Cytokine Measurement)

First, mononuclear cell fraction is isolated from blood for preparation. Heparin-added peripheral blood is diluted twofold with phosphate buffered saline (PBS) and mixed, then the mixture is layered over Ficoll-Conray solution (specific gravity: 1.077), centrifuging at 400 G is performed for 20 minutes, after which mononuclear cell fraction is collected. After washing, 10% fetal bovine serum (FBS)-added RPMI-1640 culture medium is added for preparation to provide a cell count of 1 × 10⁶. Phytohemagglutinin (manufactured by Difco) is added to 200 µl of the cell suspension thus obtained to provide a concentration of 20 µg/ml, and then the mixture is cultured using a 96 well microplate under 5% CO₂ at 37 °C for 24 hours. The cell solution thus cultured is used as the cytokine measurement sample.

### (IL-12 Measurement)

While measurement of an IL-12 amount may be conducted using known clinical or biochemical assays, a measurement kit available from R&D Systems or MBL that is based on the enzyme-linked immunosorbent assay (ELISA) is used. In this case, a measurement kit purchased from R&D Systems was used. In practice, to each well of a 96-well microplate were added 50 µl of a measuring diluent, Assay Diluent RD1F, and 200 µl of a standard solution or samples prepared by the method for preparing the aforementioned samples for cytokine measurement, after which the samples were allowed to stand still at room temperature for 2 hours for reaction. Thereafter, 200 µl of horseradish peroxidase-labeled anti-IL12 antibody was added to the solution of each well, and the wells were then allowed to stand still at room temperature for 2 hours. After removing the reaction solution from each well and washing three times, 200 µl of chromophore solution was added to each well, the wells were allowed to stand still at room temperature for 20 minutes, and 50 µl of enzyme reaction stopping solution was then added to each well. The absorbance of each sample was measured at 450 nm with Emax (from Wako Pure Chemical Industries, Ltd.) employing 550 nm as a control. The amount of IL-12 is represented in pg/ml. As used herein, the term "IL-12 production inducing capability" refers to a capability of augmenting the amount of IL-12, which is produced by peripheral blood mononuclear cell as a result of stimulation, to 7.8 pg/ml or more, or a capability of augmenting the amount of IL-12 produced to one that is more than an amount of IL-12 produced before administration of a certain substance.

### (IFNγ Measurement)

Measurement of IFNγ was conducted by enzyme immunoassay (EIA) using the IFNγ EASIA kit available from BioSource Europe SA. In practice, to each well of a 96-well microplate was added 50 µl of a standard solution or a solution obtained by diluting the aforementioned prepared sample twofold, 50 µl of HRP-labeled anti-IFNγ antibody was added to each well for reaction for 2 hours at room temperature while shaking. After removing the reaction solution from each well and washing three times, 200 µl of chromophore solution was added to each well, reaction was allowed for 15 minutes at room temperature while shaking, and 50 µl of enzyme reaction stopping solution was then added to each well. The absorbance for each well was measured at 450 nm and 490 nm with Emax (from Wako Pure Chemical Industries, Ltd.) employing 630 nm as a control. The amount of IFNγ is represented in IU/ml.

### (Measurement of Neovascularization Inhibiting Capabilities)

### (Measurement of vascular endothelial growth factor/VEGF, basic fibroblast growth factor/bFGF and neovascularization inhibitor endostatin/endostatin)

Serum concentrations were measured using commercially available kits for each enzyme linked immunosorbent assay (ELISA) (ACCUCYTE Human VEGF, ACCUCYTE Human bFGF, ACCUCYTE Human Endostatin, from Cytimmune Sciences Inc.).

### (Th2 Measurement)

Th2 denotes a proportional value of IFNγ negative and IL-4 positive cells among helper T-cells (100 %) having cell surface antigen CD4.

Blood collected from cancer patients was added to phorbol 12-myristate 13 acetate (PMA) and ionomycin in the presence of breferdin A (BFA) and stimulated for 4 hours at 37 °C. Cells in the blood were stimulated with PMA and ionomycin to produce cytokines, and trafficking of intracellular proteins to outside the cells was inhibited by BFA. To activated samples prepared in this manner was added CD4-PC5 (from Beckman Coulter Inc.) to stain CD4 on the cell surface. Next, after hemolysis and fixation with FACS Lysing Solution (Becton Dickinson), cells were permeabilized with FACS Permeabilizing Solution (Becton Dickinson). Thereafter, intracellular cytokines were stained using IFN-γ FITC/IL-4 PE (Becton Dickinson), and then measured with a flow cytometer (FACS Calibur, Becton Dickinson) for analysis.

### [Measurement of Th1/Th2 (cell) Ratio]

The Th1/Th2 cell ratio was determined according to an ordinary method by helper T(Th)-cell line three-color analytical assay using flow cytometry. Th1/Th2 shows the ratio between cells (Th1) that produce IFNγ and cells (Th2) that produce IL-4 among the helper T-cells having the cell surface antigen CD4, and it is represented as CD4×IFNγ/IL-4.

First, blood collected from cancer patients was treated for 4 hours at 37 °C with phorbol 12-myristate 13 acetate and ionomycin to stimulate cells in the blood to produce cytokines. Next, Breferdin A was added to stop the production reaction, cell surface marker CD4 was stained using an anti-CD4 antibody CD4-PC5 (Beckman Coulter), the cells were fixed and then subjected to hemolysis using FACS Lysing Solution (Nippon Becton Dickinson Co., Ltd.). Thereafter, cells were permeabilized with FACS Permeabilizing Solution (Nippon Becton Dickinson Co., Ltd.), and intracellular cytokines were stained using anti-IFNγ antibody/anti-IL-4 antibody (Fastimmune IFNγ FITC/IL-4 PE, from Nippon Becton Dickinson Co., Ltd.), and then measured and analysed with a flow cytometer (FACS Calibur, Becton Dickinson).

### (TNFα Measurement Method)

### 1. Isolation, preparation and culturing of mononuclear cell

First, heparin-added peripheral blood was diluted twofold with phosphate buffered saline (PBS) and mixed, then the mixture was layered over Conray-Ficoll solution (specific gravity 1.077) and centrifuged for 20 min at 1800 rpm, after which mononuclear cell fraction was collected. After washing, 10% fetal bovine serum (FBS)-added RPMI-1640 culture medium was added for preparation to provide a cell count of 1 × 10⁶/ml. Phytohemagglutinin (30 µg/ml) (manufactured by Difco) was added to 200 µl of the cell suspension thus obtained, and then the mixture was cultured using a 96 well microplate under 5% CO₂ at 37 °C for 24 hours. After culturing, the samples were cryopreserved until measurement.

### 2. Measurement by ELISA

A standard solution or a sample of interest was added to antibody plates that had been previously immobilized with anti-human TNFα, and allowed to react. Next, the plates were washed, and POD-labeled anti-human TNFα monoclonal antibody (enzyme-labeled antibody) was added thereto for reaction. After washing the plates again, substrate was added for enzyme reaction, and activity was read as an absorbance at a wavelength of 492 nm.

A commercially available product was used for each of the markers used in the clinical testing, and measurement values were shown in accordance with the respective recommended methods. All abbreviations shown are in accordance with common methods of presentation.

Judgment of the efficacy on patients was carried out using the following 5 judgment levels: CR (complete remission), PR (partial remission), LNC (no long-term change), SNC (no short-term change) and PD (progressive disease). Further, the rate of efficacy for each cancer type indicates the rate of CR, PR, LNC, SNC, or PD among all cases of each cancer type (for example, PR 71.4% in a total number of 7 cases of colon cancer indicates that PR was observed in 5 of the 7 cases).

### Examples

Hereunder, the present invention is described in detail using examples, however the present invention is not limited to these examples.

As a novel immunotherapy for cancer (NITC), treatments had been conducted for progressive terminal cancer cases. At the end of April 2002, effective cases of CR or PR were obtained in 35.3% of 3490 cases. This NITC is a BRM therapy in which administration of β1,3 glucan induces endogenous TNFα, IFNγ, and IL-12 to activate CTL (killer T cells), administration of α1,3 glucan activates NK and NKT cells, and oral administration of Better Shark inhibits vascularization. Patients were administered with IL-12 inducers, shark cartilage (Seishin Enterprise Co., Ltd.) and saccharides having an α1,3 structure and the like according to the recommended presecriptions for each. Further, as IL-12 inducers, ILX (Tozai Iyaku Kenkyusho, Ltd.), ILY (Seishin Enterprise Co., Ltd.), Krestin (Sankyo), Immutol (NBG) and the like were administered independently or in their combination, and roughly the same effects were obtained.

### (Example 1)

### Case 1

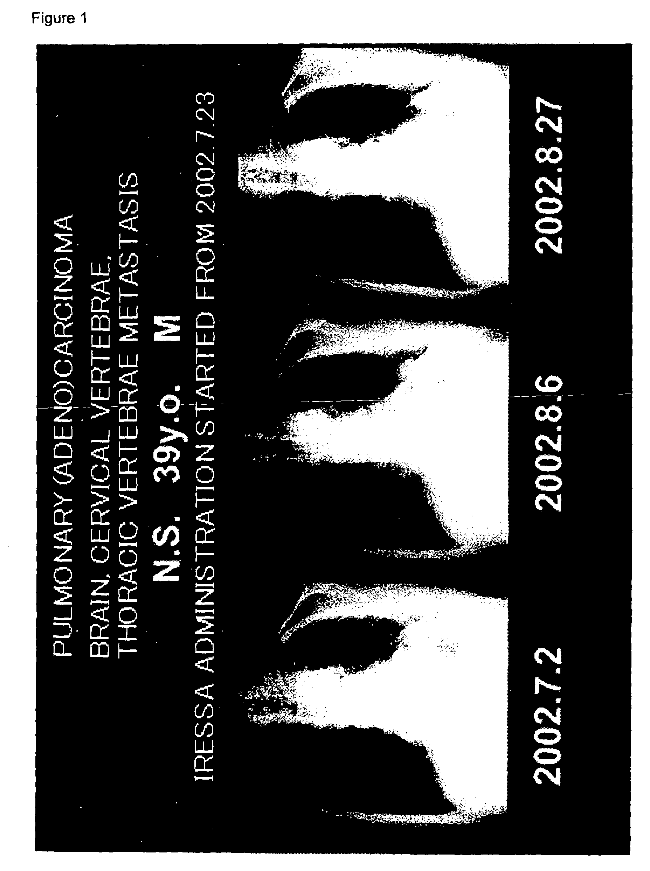

Oral administration of 250 mg of IRESSA per day was supplemented for a case (NITC PD case) of terminal pulmonary adenocarcinoma (miliary metastasis in both lungs) in which bone metastasis to cervical vertebrae, thoracic vertebrae and hip joint and brain metastasis were observed. After one and a half months carcinomatous pleural fluid and primary lung cancer had completely disappeared, bone metastasis to right hip joint, cervical vertebrae and thoracic vertebrae was cured, TNFα, IFNγ, and IL-12 were activated at levels exceeding standard values, and various tumor markers were also normalized, and the case was rated as CR (Figure 1) (Table 1).

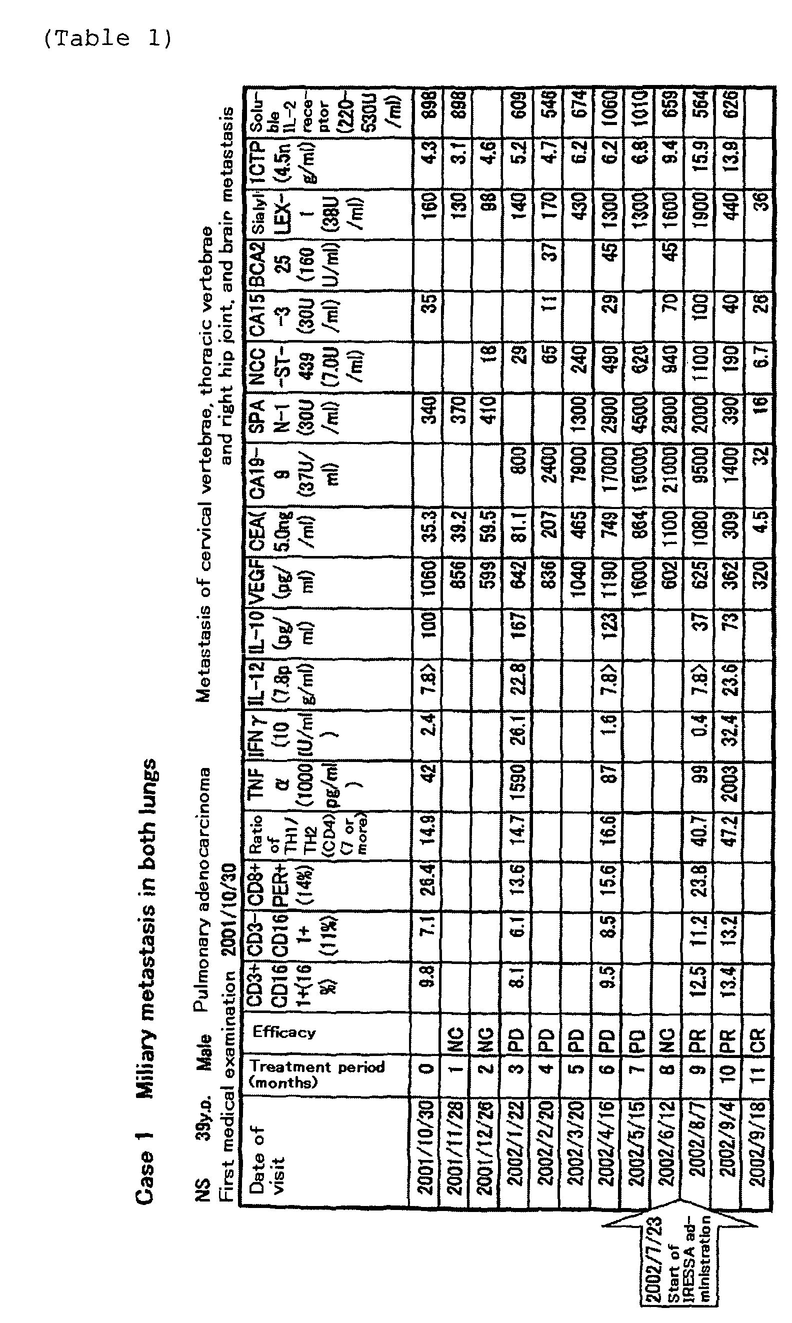

### Case 2

Supplemental administration of 250 mg/day of ZA1839 (IRESSA) was combined with NITC in a prostatic cancer case with multiple bone metastasis that was a terminal cancer case exhibiting hormone resistance, anticancer agent resistance and immunotherapy resistance. After one month, complete remission was observed for the multiple bone metastasis and the PSA value was normalized from 170 mg/ml to 4.0 ng/ml (CR judgment) (Table 2).

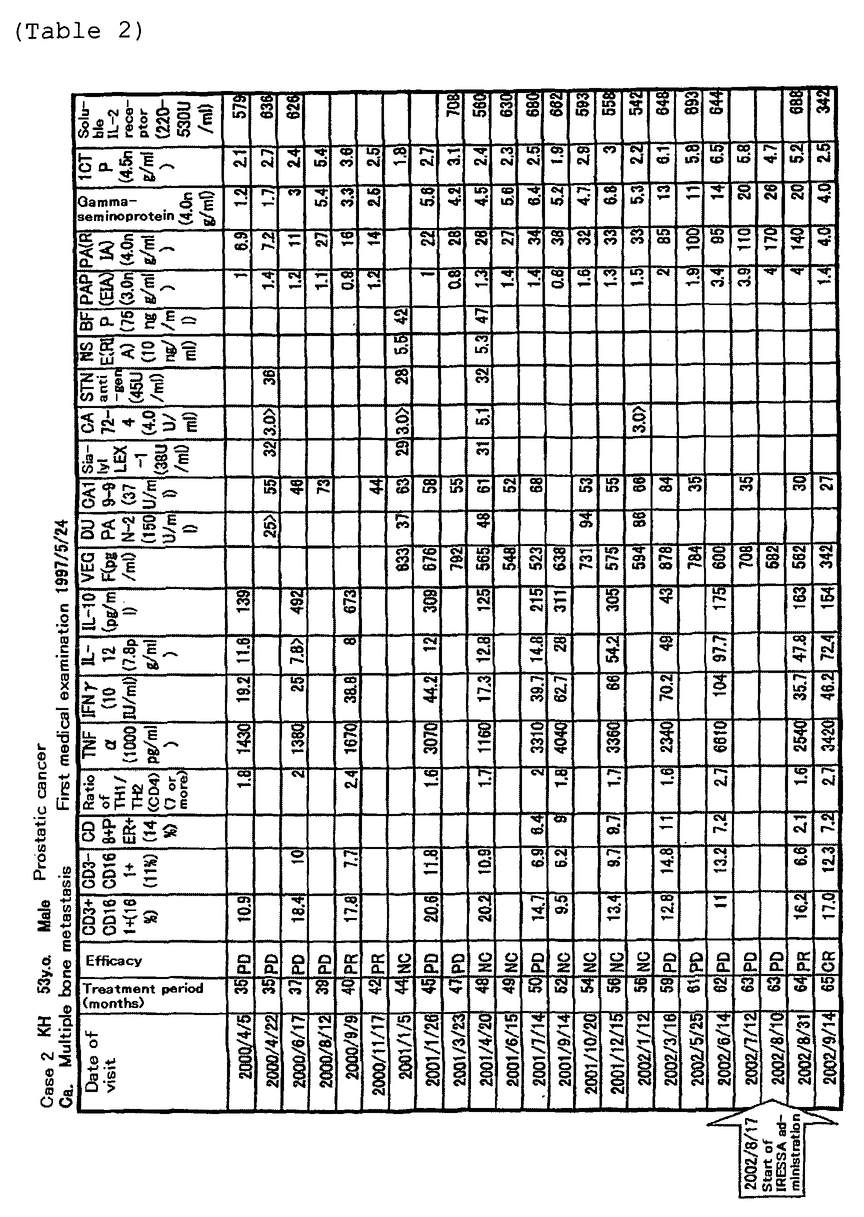

### Case 3

For a case in which miliary lung metastasis and multiple costal metastasis had been observed in both lungs in right pulmonary adenocarcinoma and respiratory distress and severe backache had been occurred, one IRESSA tablet of 250 mg/day was administered everyday from August 3, 2002, in combination with NITC.

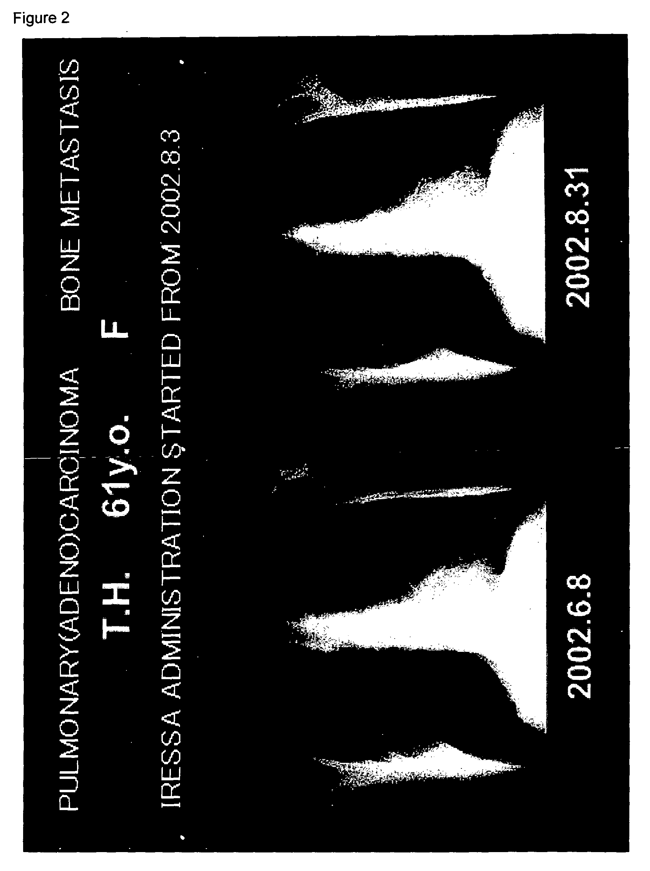

Approximately 1 month after administration began on August 31, primary focus in the right lung was reduced by half, miliary lung metastasis almost disappeared, and multiple costal metastasis disappeared. Induced production of TNFα, IFNγ, and IL-12 increased, and the tumor marker CEA decreased from 256 ng/ml prior to the treatment to 172 ng/ml, while SLX-1 decreased by more than half from 480 U/ml prior to the treatment to 140 U/ml (PR judgment) (Figure 2) (Table 3).

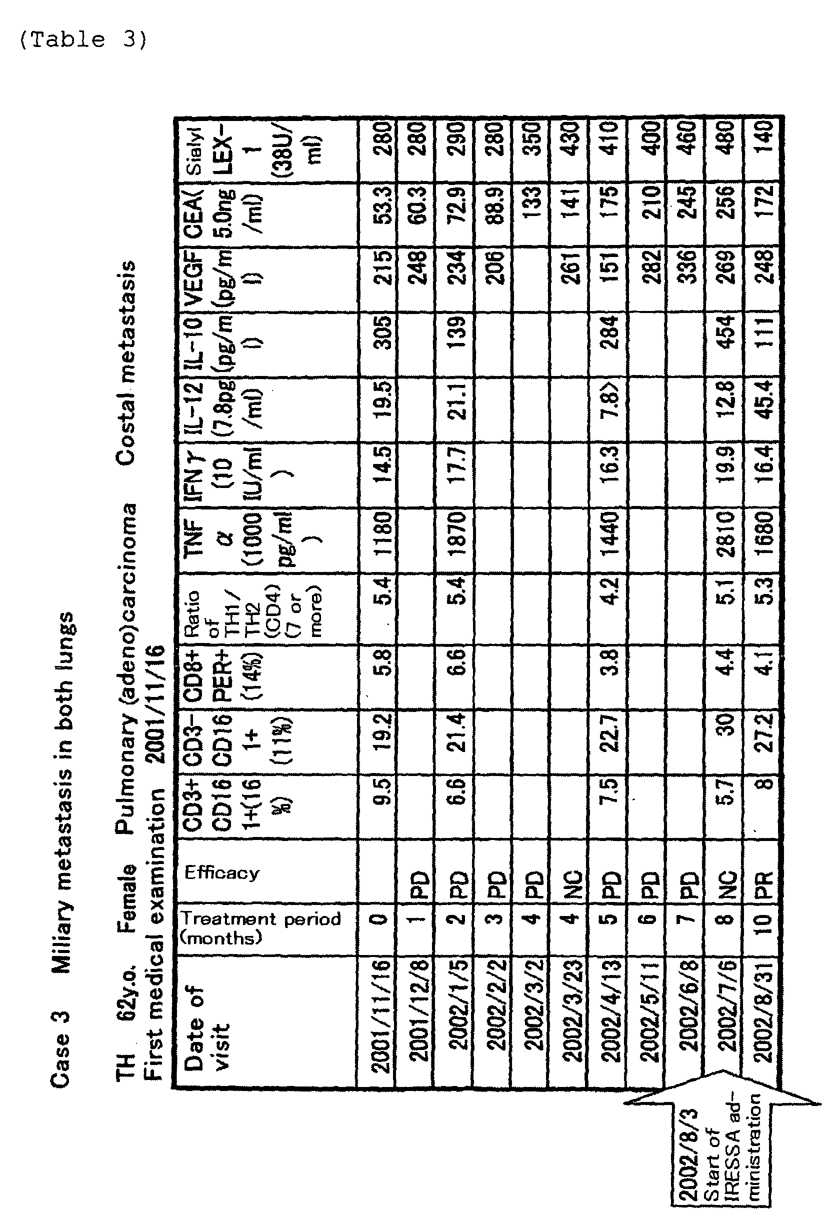

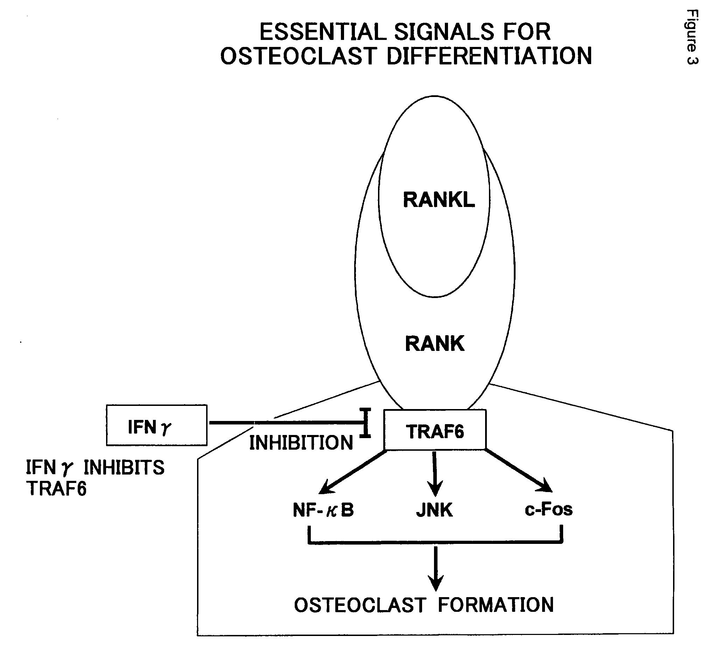

Although combined use of NITC, anticancer agents or radiation inhibits induction of IFNγ and IL-12 production, it was observed that combined use of NITC and IRESSA does not suppress production of IFNγ and IL-12, but rather tends to increase the production. It is recognized that production of Th1 cytokines is important for improving bone metastasis, and inhibits differentiation and proliferation of osteoclasts by inhibiting TRAF6. Further, IRESSA is capable of inhibiting differentiation and proliferation of osteoclasts by inhibiting signal transduction of c-fos mRNA downstream of TRAF6 (Figure 3).

IRESSA suppresses expression of c-fos mRNA to suppress the tyrosine kinase signal transduction system of EGFR and inhibit differentiation of osteoclasts. Further, differentiation of osteoclasts upstream of c-fos can be inhibited by increasing the amount of Th1 cytokines such as IFNγ and IL-12 by novel immunotherapy for cancer (NITC), and IRESSA and NITC can additionally or synergistically improve bone metastasis. In this respect, bone metastasis is inhibited by a dual system in which production of TRAF6 is suppressed in osteoclast differentiation by augumented production of Th1 cytokines through NITC, and expression of c-fos mRNA downstream of TRAF6 is inhibited by IRESSA. Accordingly, it is considered that NITC and IRESSA acted additionally or synergistically with respect to bone metastasis to improve the bone metastasis of case 1, case 2 and case 3.

While IRESSA acts either directly or indirectly in an antitumoral manner on cancer cells, NITC augments production of Th1 cytokines (TNFα, IFNγ, and IL-12) through β1,3 glucan administration to activate not only CTL but also NK or NKT cell. Meanwhile, NITC activates NK and NKT cells through α1,3 glucan administration to activate effector cells. It is known that NITC also promotes ADCC activation. That is, the molecule-targeting therapy of IRESSA and immunotherapy have been recognized to have mutually complementary actions that enhance therapeutic effects for cancer.

In treatment by IRESSA, a reduction (PR) (about 20%) in cancer cells and cessation of proliferation (NC) (about 50%) through EGFR tyrosine kinase inhibition was achieved. Combined use with NITC enables promotion of CTL activation through increased production of Th1 cytokines by β1,3 glucan administration and activation of NK and NKT cells through α1,3 glucan administration, making it possible to proliferate and activate the respective effector cells. It is also considered that it is easier for these activated CTL, NK and NKT cells to attack tumor cells on which IRESSA has previously acted to decelerate or terminate their development and growth. It has thus become possible to treat even intractable malignant tumors such as miliary lung metastasis and bone metastasis that heretofore had been considered untreatable.

### (Example 2)

### Case 4 73-year old male

On August 10, 2000, the patient's small intestine was removed due to small intestinal leiomyosarcoma, and on December 27, 2001, transcatheter arterial embolization was conducted for liver metastasis and a longitudinal contraction was observed. Thereafter, in July 2002, liver metastasis increased and peritoneal metastasis also appeared. Consequently, NITC was commenced from July 22, 2002. From August 20, 2002, administration of 400 mg of Gleevec per day was also started. Activation of Th1 cytokines was occurring on August 20, and the respective values were TNFα: 2570 pg/ml, IFNγ: 17.5 IU/ml, and IL-12: 49.8pg/ml. NK activity also exhibited a strong value [CD3(-)CD161(+): 30.60]. However, NKT cell activity was not observed [CD3(+)CD161(+)perforin(+): 29.9%]. Tumor markers exhibited high values, with Gat at 22.9 u/ml (normal value is 13. 6 or less), BFP at 93 ng/ml (normal value is 75 or less) and ICTP at 5.2 ng/ml (normal value is 4.5 or less). Combined therapy of NITC and Gleevec was continued for about 1 month, and the values of the respective immune markers measured on September 19, 2002 had increased from those at the previous measurement. The respective values were TNFα: 4322 pg/ml, IFNγ: 34.8 IU/ml, IL-12: 98.3 pg/ml, NK activity: CD3(-)CD161(+) : 35.4%, and NKT cell activity: CD3(+)CD161(+)perforin(+) : 32.4%. For the tumor markers, GAT showed a value of 18.3 u/ml (normal value is 13. 6 or less), BFP was 79 ng/ml (normal value is 75 or less) and ICTP was 4.8 ng/ml (normal value is 4.5 or less). Surprisingly, in an ultrasonic examination conducted at the same time, liver metastasis had decreased by 50% or more and tumor markers that had exhibited abnormality had all improved.

### Case 5 62-year old female Myosarcoma of the Uterus

On January 26, 2000, the patient underwent a total hysterectomy and ablation of appendages on both sides for myosarcoma of the uterus. However, intraperitoneal residual tumor was observed. From February to May 2000, the patient was administered with the anticancer agents Paraplatin, Endoxan and Therarubicin, but was rated PD. On November 15, 2001, a fist-sized tumor appeared on the left wall of the abdominal region and ablation was conducted again on January 23, 2002. Thereafter, although the patient was administered with the anticancer agent Ifomide from February to April 2002, in April 2002 metastasis was again observed on the right abdominal wall in the form of a 6×5 cm-sized uterine myosarcoma. In an ultrasonic examination on August 10, 2002, enlarged sarcomas of 103×88×81 mm and 29×30×27 mm were observed on the right abdominal wall and left abdominal wall, respectively.

From August 10, 2002, combined therapy of NITC and 400 mg/day of Gleevec was conducted. In an ultrasonic examination on September 17, 2002, the tumor on the right abdominal wall and the tumor on the left abdominal wall had decreased in size by half to 58×40×39 mm and 15×14×13 mm, respectively. Further, enhancement in ability to produce Th1 cytokines was confirmed [TNFα (4106 pg/ml), IFNγ (33.5 IU/ml) and IL-12 (80.6 pg/ml)].

Case 4 and case 5 were both cases of terminal sarcoma. In these cases, although PD was judged for treatment by NITC alone, in the extremely short period of approximately one month of combined treatment with Gleevec, a notable improvement (PR) was achieved in both cases. Cases of Gleevec administration reported up to now have reported only effective case of about 20% after 4 to 6 months of administration. However, in the present clinical cases PR was achieved in all of the cases in an extremely short period. It was presumed that combined treatment with NITC therapy, particularly IL-12 production-inducing therapy, and Gleevec exerted a synergistic effect for anticancer treatment, similarly to the aforementioned combined treatment with NITC therapy, particularly IL-12 production-inducing therapy, and IRESSA. Thus, according to the present invention, it was confirmed that combined use of a tyrosine kinase inhibitor and a Th1 cytokine production enhancer has a synergistic effect for anticancer efficacy. In this connection, although a notable suppression of Th1 cytokine values was observed in combined therapy of anticancer agents and NITC, it was confirmed that in the combined therapy of a tyrosine kinase inhibitor and NITC of the present invention, both of them increased immunological competence and acted synergistically for an antitumor action.

### (Example 3)

The therapeutic results were reviewed for 55 cases of treatment combining NITC and IRESSA including cases 1 to 5 of Examples 1 and 2 of immunotherapy for cancer. The levels of cancer progression in the 55 cases ranged from initial stage to advanced terminal stage, and the cancer types were lung cancer, cancer of the large intestinal, anal cancer, renal cancer, lingual cancer, breast cancer, gastric cancer, prostatic cancer, esophageal cancer, pancreatic cancer, pharyngeal cancer, parotid cancer, bladder cancer, cervical cancer and ovarian cancer. The period of combined treatment of NITC and IRESSA was approximately 2 to 4 months for each case. Further, similarly to Example 1, an IL-12 inducer, shark cartilage (Seishin Enterprise Co., Ltd.), and a saccharide (NK, NKT activator) having an α1,3 structure were administered to the patients in each case according to the respective recommended prescriptions. IRESSA was also administered according to the recommended prescription, for example, an oral administration of 250 mg per day, as in case 1.

### Examination of effective rate for each cancer type

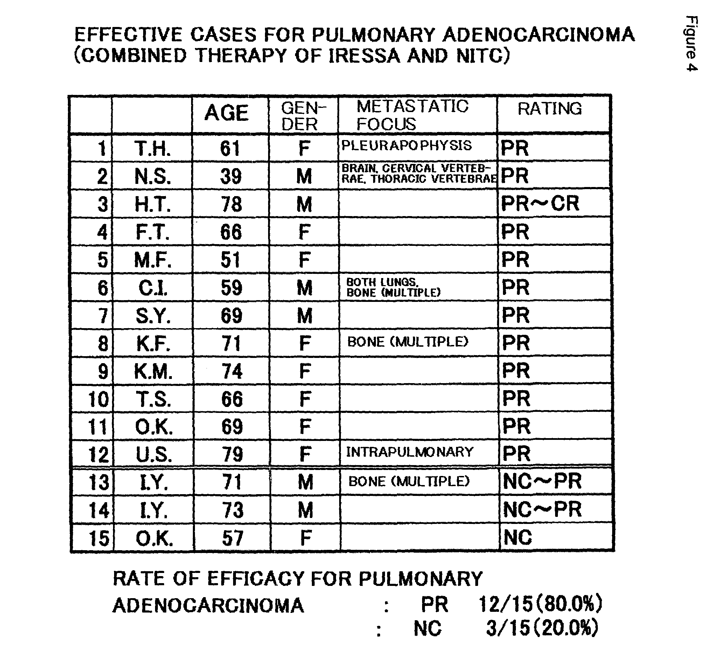

PR cases accounted for 12 out of 15 cases of pulmonary adenocarcinoma, representing a proportion of effective cases of 80%. The remaining 3 cases were rated NC. Four of the pulmonary adenocarcinoma cases were miliary lung metastasis cases. More specifically, miliary lung metastasis is a cancer with the worst prognosis among pulmonary adenocarcinoma cases, is complicated with respiratory distress, and is almost impossible to improve with the conventional therapy. It is considered that it is not possible for this kind of miliary lung metastasis case to be improved by administration of IRESSA alone. Further, while the proportion of effective cases of treating pulmonary adenocarcinoma with IRESSA alone reported up to now is around 20%, the combined use of NITC and IRESSA this time produced a surprising improvement rate of PR in 12 out of 15 cases, or 80% (Figure 4).

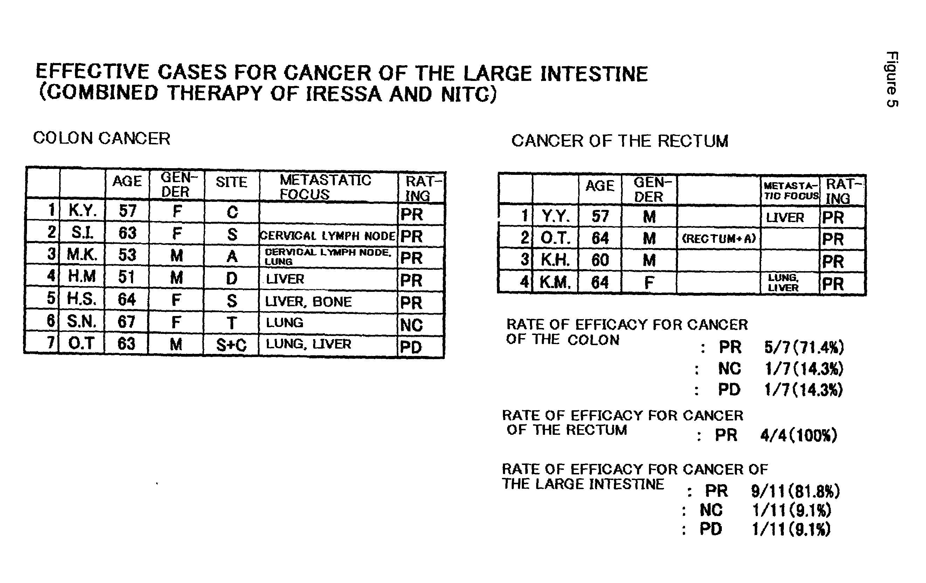

Cancer of the large intestinal is classified into cancer of the rectum and colon cancer. In general, the prognosis for cancer of the rectum is considered poor in comparison to colon cancer. The combined treatment of NITC and IRESSA resulted in a PR rating in all 4 cases of cancer of the rectum, representing an effective rate of 100%. Meanwhile, in 7 cases of colon cancer, there were 5 PR cases (71.4%), 1 NC case (14.3%) and 1 PD case (14.3%). Accordingly, there were 9 PR cases for cancer of the large intestinal, representing a surprising improvement rate of 81.8% (Figure 5).

The efficacy of combined treatment with NITC and IRESSA for malignant tumors in which PR cases were observed for cancer types other than pulmonary adenocarcinoma and colon cancer was as follows: 1 out of 2 cases of anal cancer (50%), 2 out of 2 cases of renal cancer (100%), 1 out of 1 case of lingual cancer (100%), 2 out of 4 cases of ovarian cancer (50%), 1 out of 3 cases of gastric cancer (33.3%), and 1 out of 6 cases of breast cancer (16.7%). Further, 2 out of 2 cases of prostatic cancer were NC cases, and although the PSA of the tumor markers did not decrease, pain associated with bone metastasis was improved markedly. For esophageal cancer, 2 out of 3 cases were NC (66.7%), and in each of pancreatic cancer, cancer of the larynx, and parotid cancer one case was NC with progression stopped. For both bladder cancer and cervical cancer, only PD cases were observed.

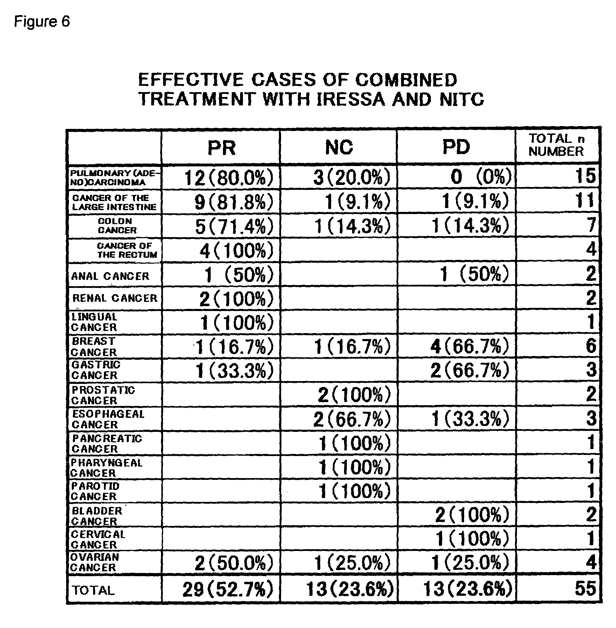

Regarding the efficacy of combined treatment with NITC and IRESSA, a trend in which the Th1 cytokines TNFα, IFNγ and IL-12 exhibited a high value was recognized in effective cases of pulmonary adenocarcinoma, large intestinal cancer (colon cancer, rectal cancer), renal cancer, lingual cancer, ovarian cancer, gastric cancer, anal cancer and breast cancer. Further, a trend of suppression of VEGF was also recognized. Meanwhile, a difference was not observed for NK cell and NKT cell between effective cases and ineffective cases. It is thus considered that these kind of notable effective cases result from the fact that combined treatment of IRESSA (trade name) and immunotherapy can particularly increase production of IFNγ and IL-12 among the Th1 cytokine (Figure 6).

Although this effect has not been currently observed for combined use of IRESSA (trade name) and anticancer agents, a therapeutic effect for various types of cancer was confirmed for the combined treatment with NITC (particularly, IL-12 inducer) and IRESSA of the present invention. In particular, the combined treatment with NITC (particularly, IL-12 inducer) and IRESSA (trade name) is effective for pulmonary adenocarcinoma, large intestinal cancer (colon cancer, rectal cancer), renal cancer, lingual cancer, ovarian cancer, gastric cancer, anal cancer, and breast cancer.

As described in the foregoing, implementation of combined treatment with NITC (particularly, IL-12 inducer) and IRESSA R of the present invention for cancer patients is an effective therapeutic method for cancer.

### (Example 4)

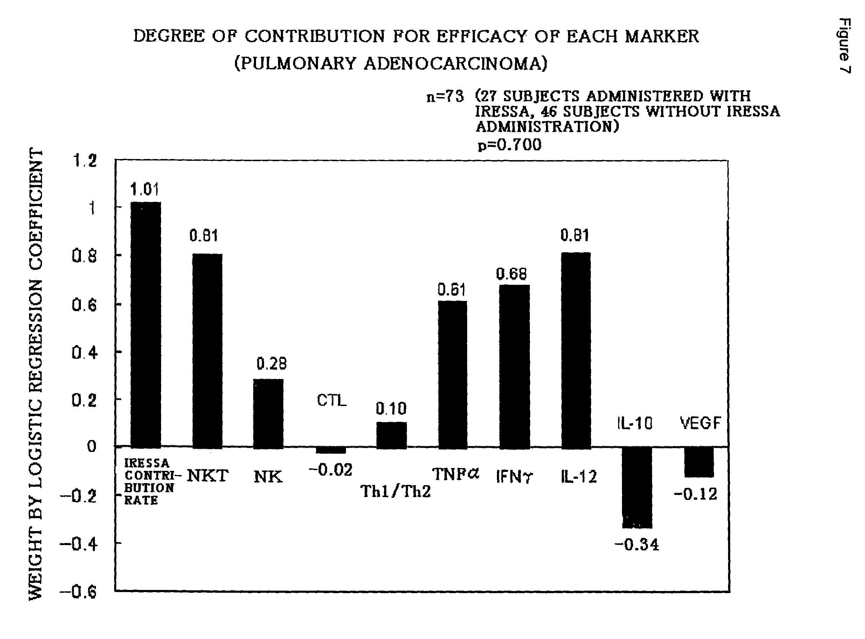

The degree of contribution of IRESSA administration and the degree of contribution (percentage) of each immunological factor were examined respectively for a total of 73 cases consisting of 46 cases of immunotherapy (NITC) alone (CR: 1 case, PR: 13 cases, LNC: 2 cases, SNC: 22 cases, PD: 8 cases) and 27 cases of combined treatment with immunotherapy (NITC) and IRESSA (250mg/day/oral administration) (CR: 2 cases, PR: 20 cases, NC: 5 cases, PD: 0 cases). Figure 7 shows the results obtained by logistic regression coefficient analysis.

When treatment for pulmonary adenocarcinoma was summerized, primarily, it was the most important whether or not IRESSA was administered, and secondly, the capability to produce IL-12 and the proportion of perforin producing cells (abbreviated as NKTP) among NKT cells were important at roughly the same level. The next significant factors were, in order, capability to produce IFNγ and capability to produce TNFα.

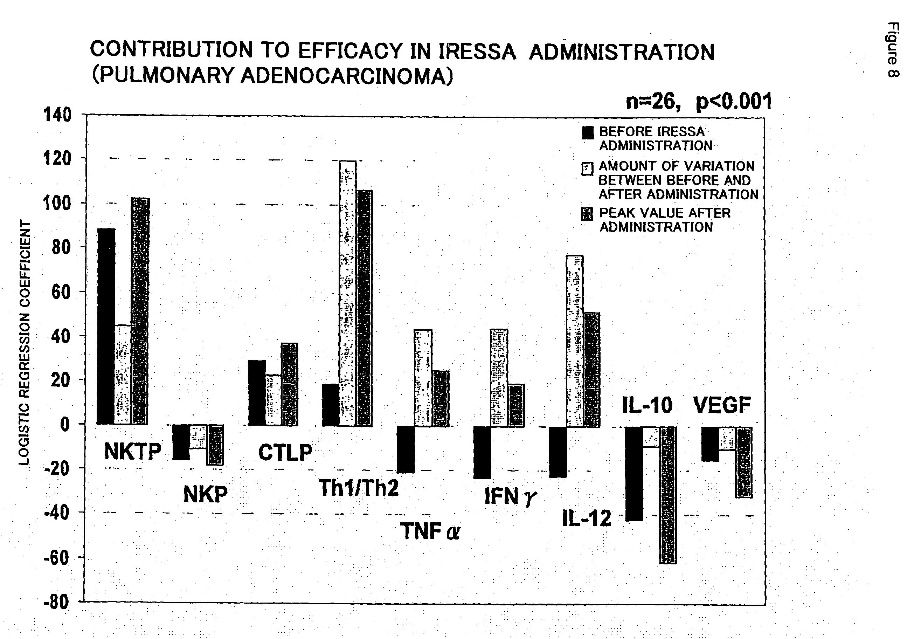

The rate of contribution of each immunological factor was examined for 26 cases of immunotherapy (NITC) and IRESSA administration. Figure 8 shows the results obtained by logistic regression coefficient analysis. The results revealed that NKTP cells (perforin positive cells among NKT cells) showed the most important significance prior to IRESSA administration. It was considered that an important point for deciding the treatment policy of pulmonary adenocarcinoma patients was judging whether or not it was possible to discriminate between an effective group and ineffective group for the present therapeutic method by means of differences in the immunological competence of patients prior to IRESSA administration.

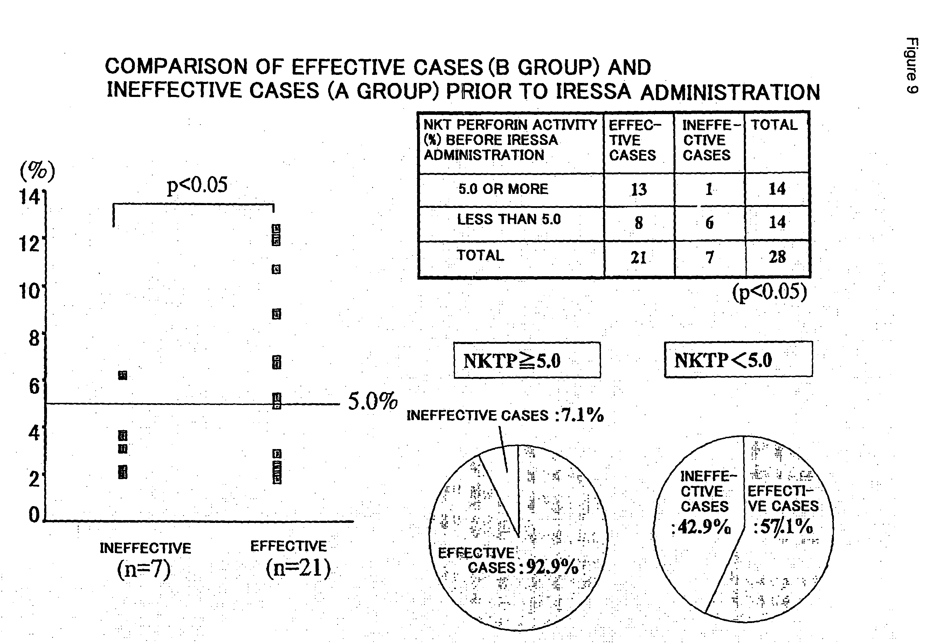

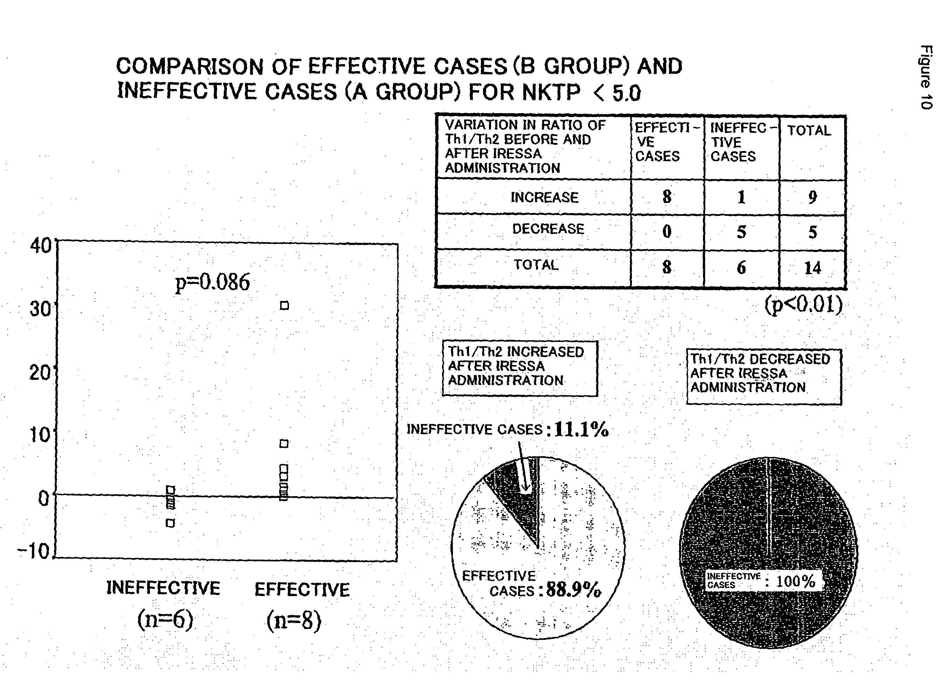

Therefore, first the proportion of NKTP cells (proportion of NKTP cells among total lymphocytes) prior to IRESSA administration was verified. The results are shown in Figure 9. When 5.0% was taken as the cutoff value for the NKTP cell count (proportion), 92.9% of cases were rated effective. However, 57.1% of cases for which the NKTP proportion was less than 5% were effective cases, and these cases would be excluded from the effectiveness judgment. It was found that these cases where the NKTP proportion was less than 5% could be judged as effective by means of the ratio of Th1/Th2. More specifically, as is apparent from Figure 8, since the Th1/Th2 ratio varies between before and after IRESSA administration with an extremely high value, cases where the NKTP proportion was less than 5% were analyzed using this indicator. As shown in Figure 10, it was found that effective cases could be judged at a rate of 88.9% when the Th1/Th2 ratio increased after IRESSA administration. All cases (100%) in which the Th1/Th2 ratio decreased after IRESSA administration were ineffective cases.

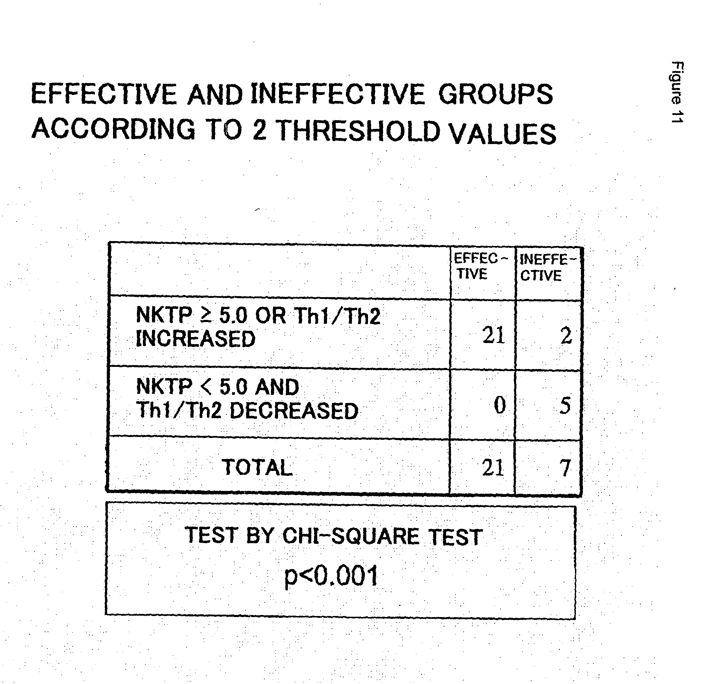

Summarizing the above results, it is suggested that if the NKTP value is 5% or more the efficacy of combined treatment of IRESSA and NITC is high, and even if the NKTP value is less than 5%, there is a high possibility that efficacy can be expected if the Th1/Th2 ratio increases after IRESSA administration. Thus, it was found from the analysis results of this example that for cases combining administration of NITC and IRESSA, if the NKTP value before IRESSA administration is 5% or more or if the Th1/Th2 ratio increases after IRESSA administration, combined administration of NITC and IRESSA can be judged as effective for a significantly high proportion of these cases (p<0.001) (Figure 11).

### (Example 5)

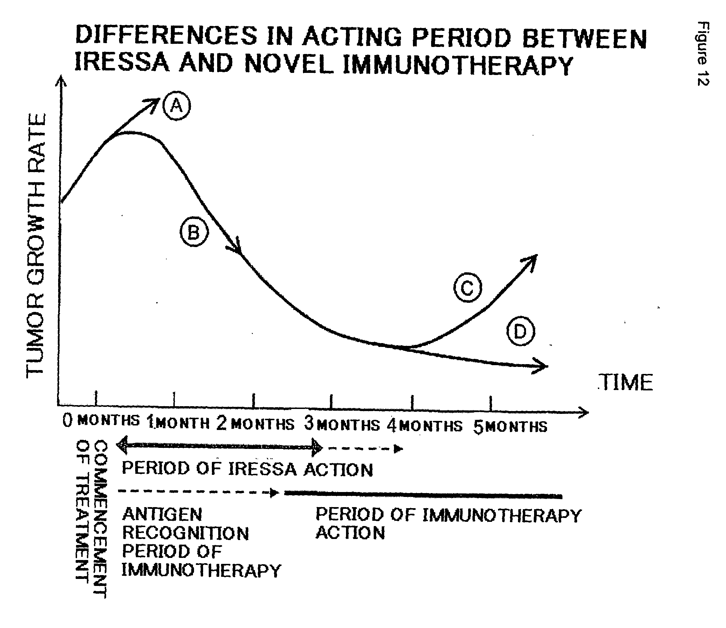

Additional usefulness of the combined treatment of immunotherapy (NITC) and IRESSA was analyzed by means of the clinical cases of Example 4. As shown by the analysis results in Figure 12, it was found that after one month of IRESSA administration the cases divided into B type in which tumor growth was suppressed, and A type in which IRESSA administration produced no effect. Further, it was found that within 6 months the B type cases in which symptoms had improved further divided into C type cases that showed recurrence and relapse and D type cases with no recurrence. It was considered that which type (A or B type, C or D type) a case would be predicted to follow at the respective branching points would be an effective indicator for immunological therapy for cancer. Also, if it were possible to predict, by measuring each immunological factor of a patient between several weeks to 5 months after IRESSA administration, preferably between 1 to 3 months, whether continuation of the combined therapy of immunotherapy (NITC) and IRESSA thereafter would be effective, it would be an effective indicator for immunological therapy for cancer. Therefore, immunological characteristics (tumor markers) of the hosts were examined to clarify factors for predicting the respective types.

The results showed that, with respect to whether a case would be A type or B type, if the NKTP value prior to IRESSA administration was 5.0% or more the case would become a B type, and even if the NKTP value was less than 5.0% the case would move in the direction of B type if the Th1/Th2 ratio increased after IRESSA administration (Figures 8 to 11). Further, it was found that if the NKTP value was less that 5% and the Th1/Th2 ratio decreased, the case would become A type and IRESSA would not exert an effect.

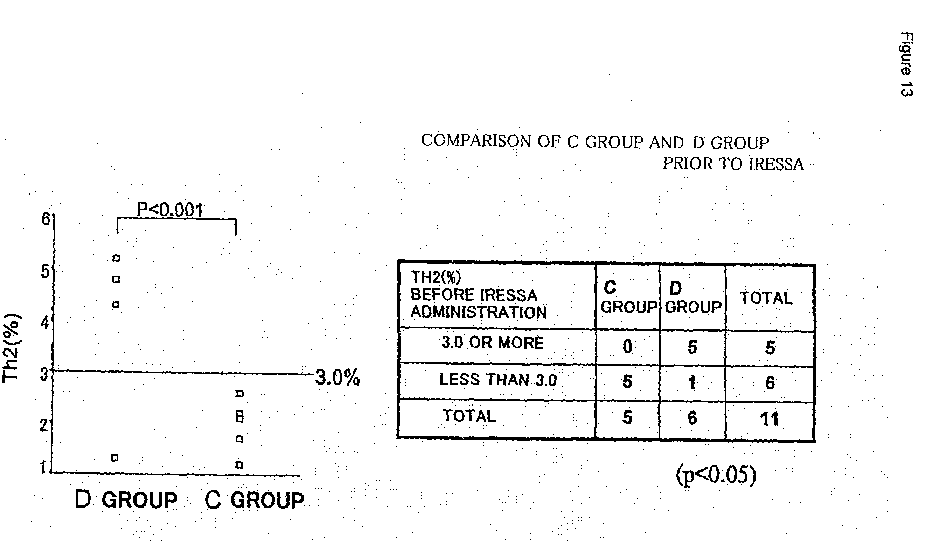

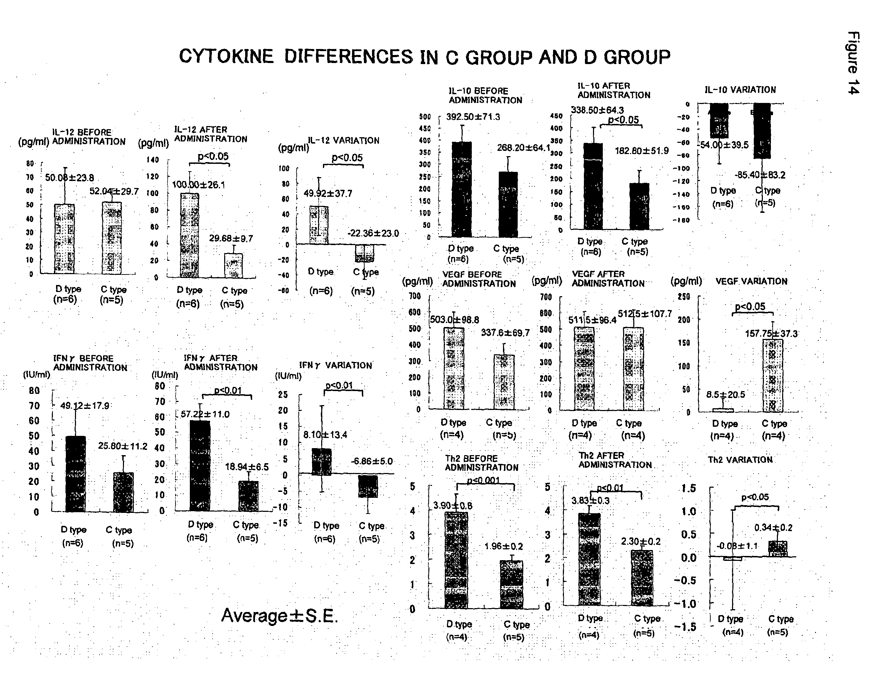

With respect to whether a case would be C type or D type, the results shown in Figure 13 revealed that if the proportion of the Th2 value exceeded 3% the case followed the direction of D type, while if the Th2 value was less than 3% the case followed the direction of C type with recurrence and relapse. However, it was found that for moving to a D type, it was important that the values of IL-12 and INFγ after IRESSA administration did not decrease even after IRESSA administration (Figure 14).

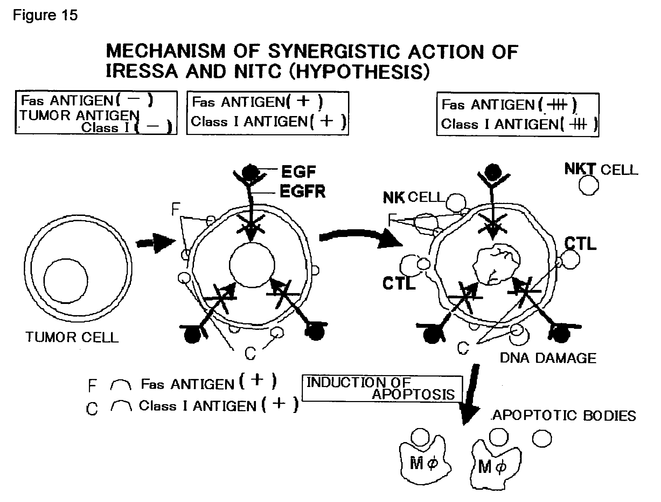

Differences in the antitumor actions between IRESSA and NITC are illustrated in the schema of Figure 15 based on the foregoing analysis results.

A cancer cell can grow remarkably without its antigens presented. However, the more intense the cell grows, the greater the amount of EGFR that should be present. IRESSA blocks intracellular signals with an inhibitory action for tyrosine kinase in the signal transduction system of the EGFR. As a result, the nucleus of the cancer cell falls into apoptosis, whereby impairment of the nucleus occurs and FAS antigen or a tumor antigen, Class I antigen or Class II antigen, is presented on the surface of the cancer cell. As a result, CTL cell (killer T cell) or NK cell recognizes the antigen, and immunocytes target the cancer cell to attack and englobe the cancer cell that has become an apoptotic body.

Accordingly, as shown in Figure 15, in combined therapy with IRESSA and NITC, first IRESSA blocks signal transduction of EGFR that prompts growth of the cancer cell. It is considered that there is a lapse of time after which immunocytes are activated to attack the cancer.

NKT perforin activity and Th1 cytokines are important for IRESSA action. Further, continuing production of IFNγ and IL-12 after administration is important for the temporary contraction of a tumor and the maintenance of that effect. It is also presumed that the Th2 immune system is necessary at that time.

### Industrial Applicability

According to the above Examples, it was found that combined use of a tyrosine kinase inhibitor and an IL-12 inducer (Th1 cytokine-production enhancement) has a synergistic effect in cancer therapy, marking a landmark achievement in cancer therapy.

## Claims

1. A therapeutic agent for cancer, wherein a tyrosine kinase inhibitor and an IL-12 inducer are used in combination.

2. The therapeutic agent for cancer according to claim 1, wherein the tyrosine kinase inhibitor has a selective targeting action on at least one receptor selected from the group consisting of the following 1) to 7):
1) HER2/neu; 2) HER3; 3) HER4; 4) c-kit; 5) PDGFR; 6) bcr-abl; and 7) EGFR.

3. The therapeutic agent for cancer according to claim 1, wherein the tyrosine kinase inhibitor has an action with EGFR or c-kit selectively targeted.

4. The therapeutic agent for cancer according to any one of claim 1 to 3, wherein the IL-12 inducer is a substance having a β1,3-1,6 glucan structure.

5. The therapeutic agent for cancer according to claim 4, wherein the IL-12 inducer is a yeast-derived ingredient or an ingredient derived from mushroom mycelium that has a β1,3-1,6 glucan structure.

6. The therapeutic agent for cancer according to any one of claim 1 to 5, which is used in no combination with a chemotherapeutic agent for cancer and a radiation therapy.

7. The therapeutic agent for cancer according to any one of claim 1 to 6, which is used in combination with a substance that selectively acts on NKR-P1 of NKT cell to cause activation of NKT cell.

8. The therapeutic agent for cancer according to any one of claim 1 to 7, which is used in combination with a substance having neovascularization inhibiting capabilities.

9. The therapeutic agent for cancer according to any one of claim 1 to 8, wherein a treatment that combines use of a tyrosine kinase inhibitor and an IL-12 inducer is carried out employing either one of the following 1) and 2) as a marker:
1) an NKTP value before administration showing a measurement value of 5% or more;
2) a Th2 value before administration showing a measurement value of 3% or more.

10. The therapeutic agent for cancer according to any one of claim 1 to 9, wherein a Th1/Th2 ratio that shows an increased measurement value after several months of administration of IRESSA in comparison to a value before administration of IRESSA is taken as a marker for continuation of the combined treatment.

11. The therapeutic agent for cancer according to claim 10, wherein an NKTP value before administration shows a measurement value below 5%.

12. The therapeutic agent for cancer according to claim 9, wherein a marker for continuation of the combined treatment is that measurement values of IL-12 and INFγ after several months of administration of IRESSA have not decreased in comparison with measurement values thereof before administration of IRESSA.

13. The therapeutic agent for cancer according to any one of claim 1 to 12, wherein the therapeutic agent for cancer is a therapeutic agent for pulmonary adenocarcinoma.

14. A therapeutic method for cancer that uses the therapeutic agent for cancer according to any one of claim 1 to 13.
